(19) Europäisches Patentamt
European Patent Office
Office européen des brevets

(11) Veröffentlichungsnummer: **0 151 393**
**B1**

(12) **EUROPÄISCHE PATENTSCHRIFT**

(45) Veröffentlichungstag der Patentschrift: **06.06.90**

(21) Anmeldenummer: **85100173.5**

(22) Anmeldetag: **09.01.85**

(51) Int. Cl.$^5$: **C 09 B 57/00** // C09B57/04, C07D487/04

# (54) Azinpigmente, Verfahren zu ihrer Herstellung sowie ihre Verwendung.

(30) Priorität: **18.01.84 DE 3401574**

(43) Veröffentlichungstag der Anmeldung:
**14.08.85 Patentblatt 85/33**

(45) Bekanntmachung des Hinweises auf die Patenterteilung:
**06.06.90 Patentblatt 90/23**

(84) Benannte Vertragsstaaten:
**CH DE FR GB LI**

(56) Entgegenhaltungen:
**EP-A-0 101 954**

**CHEMICAL ABSTRACTS, Band 86, Nr. 10, März 1977, Seite 83, Zusammenfassung Nr. 56755t, Columbus, Ohio, US; & JP-A-76 123 224 (YAMAMOTO SYNTHETIC CHEMICAL CO., LTD) 27-10-1976**

**CHEMICAL ABSTRACTS, Band 85, Nr. 24, Dezember 1976, Seite 72, Zusammenfassung Nr. 178985t, Columbus, Ohio, US; & JP-A-76 43 800 (TEKKOSHA CO., LTD) 14-04-1976**

(73) Patentinhaber: **BAYER AG**
**D-5090 Leverkusen 1 Bayerwerk (DE)**

(72) Erfinder: **Rolf, Meinhard, Dr.**
**Berta-von-Suttner-Strasse 24**
**D-5090 Leverkusen 1 (DE)**
Erfinder: **Neeff, Rütger, Dr.**
**Berta-von-Suttner-Strasse 22**
**D-5090 Leverkusen 1 (DE)**

**EP 0 151 393 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Aus der EP—A 0 101 954 sind Isoindolazine der Struktur

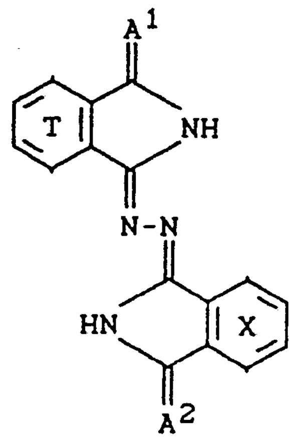

bekannt, wobei die mit X und T bezeichneten Ringe substituiert sein können und $A^1$ und $A^2$ für den zweiwertigen Rest einer Verbindung mit zwei austauschbaren Wasserstoffatomen an einem C- oder N-Atom stehen.

Die Erfindung betrifft Azinpigmente der Struktur

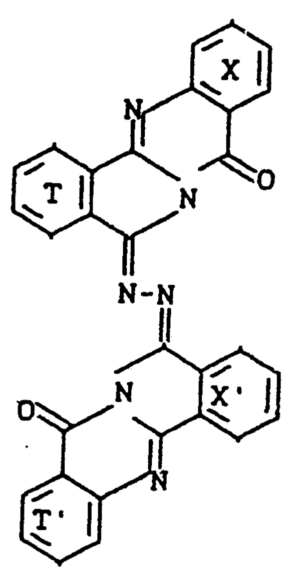

(I)

Verfahren zu ihrer Herstellung und ihre Verwendung.

In der Formel (I) können die mit X, X', T und T' bezeichneten Ringe durch jeweils 1, 2, 3 oder 4 Substituenten aus der Reihe Halogen, insbesondere Chlor und Brom; $C_1$—$C_6$-Alkyl, insbesondere Methyl; Aryl, insbesondere Phenyl, das seinerseits durch Halogen wie Chlor und Brom, $C_1$—$C_6$-Alkyl wie Methyl, Acylamino wie Acetylamino oder Benzoylamino, Nitro, Carboxy oder Carbamoyl substituiert sein kann; $C_1$—$C_6$-Alkoxy, insbesondere Methoxy; Acylamino, insbesondere Acetylamino und Benzoylamino; Carboxy; Nitro oder Carbamoyl. Die Ringe X, X', T und T' können darüber hinaus durch eventuell substituierte anellierte Benzoringe zu Naphthalinsystemen erweitert sein, wobei die Naphthalinringe durch Halogen wie Chlor und Brom, $C_1$—$C_6$-Alkyl wie Methyl, Acylamino wie Acetylamino oder Benzoylamino, Nitro, Carboxy oder Carbamoyl substituiert sein können.

Bevorzugt sind symmetrische Azinpigmente der Formel

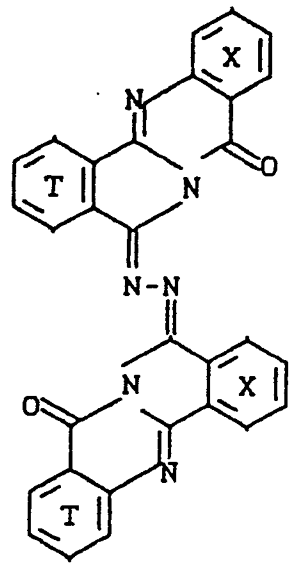

(II)

in der die mit X und/oder die mit T markierten Ringe durch 1, 2, 3 oder 4-Chlor- oder Bromatome substituiert sind.

Weiterhin bevorzugt sind symmetrische Azinpigmente der Formel (II), bei der die mit X und/oder die mit T markierten Ringe einen Substituenten aus der Reihe Methyl, Methoxy, Acetylamino, Benzoylamino, Carboxy, Carbamoyl oder Nitro tragen.

Weiterhin bevorzugt ist das symmetrische Azinpigment der Formel

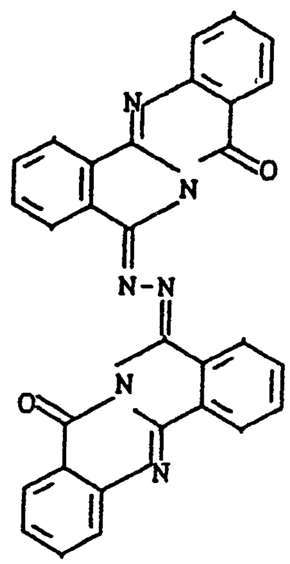

(III)

Die neuen Azinpigmente der Formel (I) sind dadurch erhältlich, daß man eine Verbindung der Formel

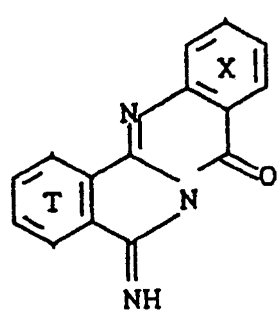

(IV)

mit einer Verbindung der Formel

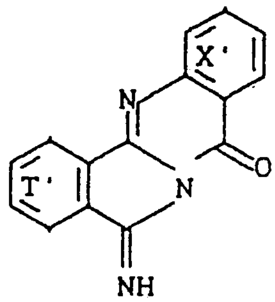

(IV a)

in Gegenwart von Hydrazin oder einer Hydrazin liefernden Verbindung wie Hydrazinhydrat oder einem Hydraziniumsalz umsetzt. In den Formeln (IV) und (IVa) haben T, T', X und X' die zu Formel (I) angegebenen Bedeutungen.

Dazu arbeitet man zweckmäßig in Wasser oder im organischen Lösungsmittel bei schwach sauren Bedingungen und erhöhter Temperatur, bevorzugt bei 50 bis 180°C. Geeignete organische Lösungsmittel sind Alkohole wie Methanol, Ethanol, Amylalkohol oder Glykolmonoalkylether; Aromaten wie Chlorbenzol, Nitrobenzol, Toluol; amidische Lösungsmittel wie Formamid, Dimethylformamid, N-Methylpyrrolidon oder Säuren wie Ameisensäure oder Essigsäure.

Geeignete Säuren zur Herstellung der sauren Bedingungen sind anorganische wie Salzäure, Schwefelsäure oder Phosphorsäure bzw. organische wie Ameisensäure, Essigsäure, Chloressigsäure, Dichloressigsäure, Oxalsäure, Benzolsulfonsäure oder p-Toluolsulfonsäure.

Anstelle der Zwischenprodukte der Formeln (IV) und (IVa) können auch deren Acylierungsprodukte der Formeln (V) und (Va) eingesetzt werden.

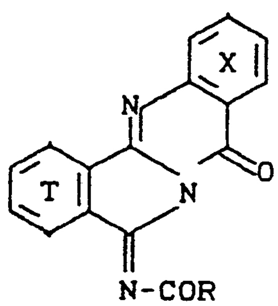

(V)

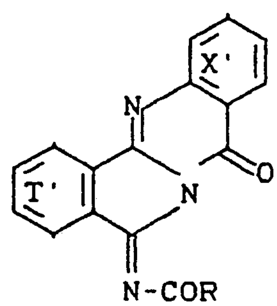

(V a)

In den Formeln (V) und (Va) haben T, T', X und X' die zu Formel (I) angegebenen Bedeutungen; R bezeichnet Alkyl, vorzugsweise $C_1$—$C_6$-Alkyl, insbesondere Methyl oder Aryl, insbesondere Phenyl.

Die Verbindungen der Formel (IV) sind durch Umsetzung eines Isatosäureanhydrids der Formel (IV)

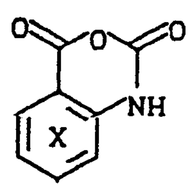

(VI)

oder eines Anthranilsäureesters oder -amids der Formel (VII)

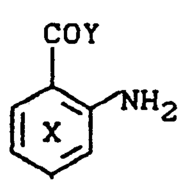

(VII)

mit mindestens einem Mol eines Aminoiminoisoindolenins der Formel (VIII)

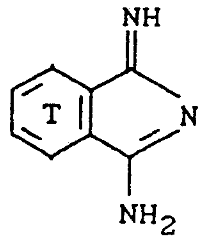

(VIII)

in einem inerten organischen Lösungsmittel, z.B. Ethanol, bei erhöhter Temperatur, vorzugsweise bei 50 bis 100°C, erhältlich.

In den Formeln (VI), (VII) und (VIII) haben T und X die zu Formel (I) angegebenen Bedeutungen.

Y in Formel (VII) bezeichnet —$NH_2$ oder —OR', wobei R' für den Kohlenwasserstoffrest eines Esters, vorzugsweise für $C_1$—$C_6$-Alkyl, steht.

Ganz analog erhält man die Zwischenprodukte der Formel (IVa).

Erfindungsgemäß einsetzbare Verbindungen, denen die Formeln (IV) und (IVa) zugeschrieben wurden, sind aus der Literatur bekannt (Angew. Chem. *68*, 135 (1956)).

Ein weiteres Herstellungsverfahren für die Pigmente der Formel (I) besteht in der Kondensation eines Hydrazons der Formel

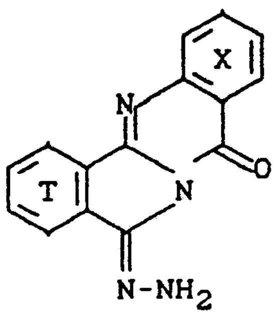

(IX)

mit einem Hydrazon der Formel

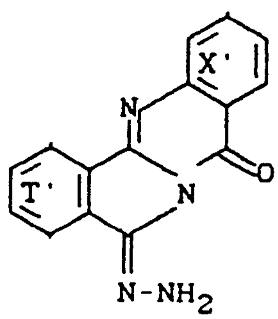

(IX a)

im sauren Medium, wobei unter Abspaltung eines Mols Hydrazin die Pigmente der Formel (I) gebildet werden. Man arbeitet dabei zweckmäßig in organischen Lösungsmitteln bei erhöhter Temperatur, vorzugsweise bei 50 bis 180°C. Die vorzugsweise verwendbaren Lösungsmittel sowie die zur Erzielung des sauren Mediums vorzugsweise eingesetzten Säuren entsprechen den für die Umsetzung der Verbindungen der wahrscheinlichen Formeln (IV) und (IVa) genannten.

In den Formeln (IX) und (IXa) haben T, T', X und X' die zu Formel (I) angegebenen Bedeutungen.

Die Verbindungen der Formeln (IV) und (IVa) bzw. (V) und (Va) bzw. (IX) und (IXa) können im beliebigen Verhältnis zueinander umgesetzt werden. Vorzugsweise handelt es sich um gleiche Verbindungen, deren Umsetzungen zu den Symmetrischen Azinpigmenten der Formel (II) führen.

Die Verbindungen der Formel (I) fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis. Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigmentes können durch Variation der Nachbehandlung beeinflußt werden.

Die Farbmittel der Formel (I) eignen sich aufgrund ihrer Licht- und Migrationsechtheit für die verschiedensten Pigmentapplikationen. Sie können zur Herstellung von sehr echt pigmentierten Systemen, wie Mischung mit anderen Stoffen, Zubereitungen, Anstrichmitteln, Druckfarben, befärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden. Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen.

Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylestern, Methacrylestern, Acrylnitril, Acrylamid, Butadien, Styrol sowie Polyurethane und Polycarbonat. Die mit den beanspruchten Produkten pigmentierten Stoffe können in beliebiger Form vorliegen.

Die Pigmente der Formel (I) sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Beispiel 1

a) In einem Gemisch aus 500 ml Nitrobenzol und 20 ml Eisessig werden 50 g des Umsetzungsproduktes von Aminoimino-isoindolenin mit Isatosäureanhydrid bei 110°C mit 5,5 g Hydrainhydrat versetzt. Man rührt 5 Stunden bei 110°C und 3 Stunden bei 150° nach, saugt heiß ab und erhält 26 g eines gelben Pigmentes der Formel

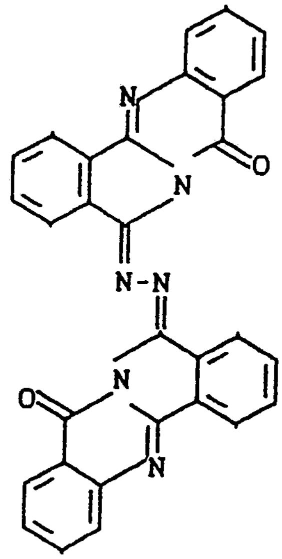

(I)

Schmelzpunkt: 360°C

Massenspektrum m/e (rel. Intensität):

492 $M^+$ (100%),

404 (54), 435 (15), 219 (30), 204 (50), 90 (60), 76 (45).

IR-Spektrum ($cm^{-1}$): 3400, 1700, 1640, 1580, 1450, 1320, 1240, 1120, 930, 760, 690.

b) In ein Gemisch aus 100 ml Dimethylformamid und 5 ml 96%iger Schwefelsäure trägt man 10 g des Hydrazons der Formel

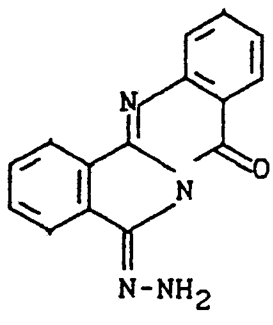

ein und rührt 30 Minuten bei 130°C. Nach Absaugen und Trocknen erhält man so 4 g eines Gelbpigmentes, das mit dem nach Beispiel 1 a erhaltenen identisch ist.

Analog Beispiel 1 a erhält man bei Einsatz substituierter Ausgangsmaterialien entsprechende Azinpigmente mit den in der Tabelle angegebenen Farbtönen.

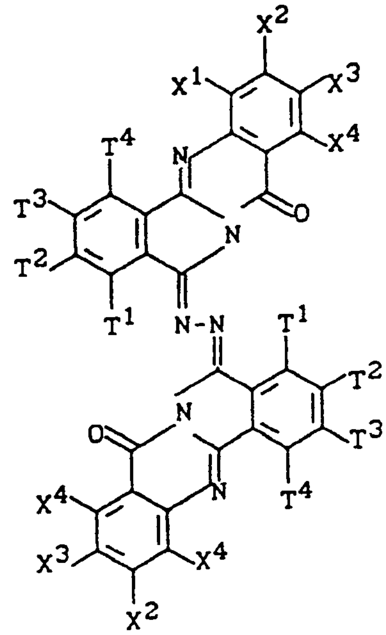

| Beispiel | $T^1$ | $T^2$ | $T^3$ | $T^4$ | $X^1$ | $X^2$ | $X^3$ | $X^4$ | Farbton |
|---|---|---|---|---|---|---|---|---|---|
| 2 | H | $C_6H_5$ | H | H | H | H | H | H | rotstichig Gelb |
| 3 | Cl | Cl | Cl | Cl | H | H | H | H | Orange |
| 4 | H | H | H | H | H | Cl | H | H | grünstichig Gelb |
| 5 | H | H | H | H | H | H | Cl | H | grünstichig Gelb |
| 6 | H | H | H | H | H | H | H | Cl | grünstichig Gelb |
| 7 | H | H | H | H | Cl | H | Cl | H | grünstichig Gelb |
| 8 | H | H | H | H | Br | H | Br | H | Gelb |
| 9 | H | H | H | H | Cl | Cl | Cl | Cl | grünstichig Gelb |
| 10 | H | H | H | H | H | $NO_2$ | H | H | Gelb |
| 11 | H | H | H | H | H | H | $NO_2$ | H | Gelb |
| 12 | H | H | H | H | H | $NHCOCH_3$ | H | H | Gelb |
| 13 | H | H | H | H | H | $CH_3$ | H | H | Gelb |
| 14 | H | H | H | H | H | $OCH_3$ | H | H | Orange |
| 15 | H | H | H | H | H | $CONH_2$ | H | H | Gelb |
| 16 | Cl | Cl | Cl | Cl | Cl | Cl | Cl | Cl | Orange |

Beispiel 17

Analog Beispiel 1 a erhält man bei Umsetzung eines Gemisches aus 5 g (X) und 7,8 g (XI) ein gelbes Azinpigment,

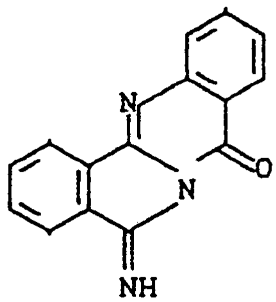
(X)

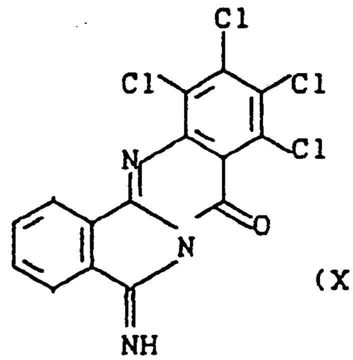
(XI)

in dem die beiden Komponenten (X) und (XI) gemischt vorliegen.

Beispiel 18—19

Analog Beispiel 1 a erhält man bei Einsatz der Zwischenprodukte (XII) bzw. (XIII)

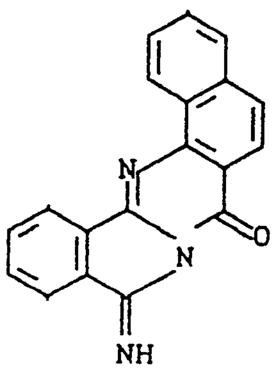
(XII)

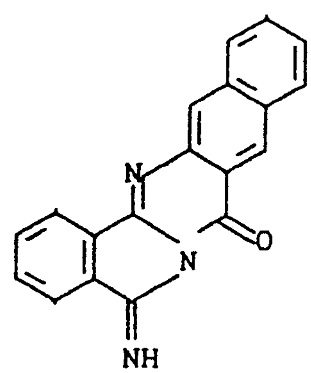
(XIII)

die entsprechenden dimeren Azinpigmente, die braune Farbtöne zeigen.

Beispiel 20 (Anwendungsbeispiel)

8 g feingemahlenes Pigment gemäß Beispiel 1 a werden in 92 g eines Einbrennlackes folgender Zusammensetzung dispergiert:

13% Alkydharz
15% Melaminharz
5% Glykolmonomethylether
34% Xylol
13% Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden.

Nach erfolgter Dispergirung wird der pigmentierte Lack aus Papier-, Glas-, Kunststoff- oder Metallfolien aufgetragen und 30 Minuten bei 130°C eingebrannt. Die Lackierungen besitzen sehr gute Licht- und Wetterbeständigkeit sowie gute Überlackierechtheit.

Beispiel 21 (Anwendungsbeispiel)

0,2 g Pigment nach Beispiel 1 a werden mit 100 g Polyethylen-, Polypropylen- oder Polystyrolgranulat gemischt. Die Mischung kann entweder bei 220 bis 280°C direkt in einer Spritzgußmaschine verspritz oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgußmaschine verspritzt. Die gelben Formlinge besitzen sehr gute Licht- und Migrationsechtheit. In ähnlicher Weise können bei 280 bis 300°C, gegebenenfalls unter Stickstoffatmosphäre, synthetische Polyamide aus Caprolactam oder

Adipinsäure und Hexamethylendiamin oder die Kondensate aus Terephthalsäure und Ethylenglykol gefärbt werden.

Beispiel 22 (Anwendungsbeispiel)

Mit einer Druckfarbe, hergestellt durch Anreiben von 35 g Pigment nach Beispiel 1a und 65 g Leinöl und Zugabe von 1 g Siccativ (Co-Naphthenat, 50 %ig in Testbenzin) werden gelbe Offset-Drucke hoher Brillanz und Farbstärke und sehr gute Licht- und Lackierechtheiten erhalten. Verwendung dieser Druckfarbe in Buch-, Licht-, Stein- oder Stahlstichdruck führt zu gelben Drucken ähnlicher Echtheiten. Verwendet man das Pigment zur Färbung von Bleckdruck- oder niedrigviskosen Tiefdrucktinten, erhält man orangefarbene Drucke ähnlicher Echtheiten.

**Patentansprüche**

1. Azinpigmente der Struktur

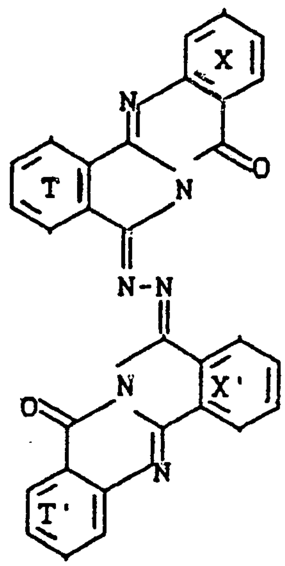

(I)

wobei die mit X, X', T und T' bezeichneten Ringe durch jeweils 1, 2, 3 oder 4 Substituenten aus der Reihe Halogen, insbesondere Chlor und Brom; $C_1$—$C_6$-Alkyl, insbesondere Methyl; Aryl, insbesondere Phenyl, das seinerseits durch Halogen wie Chlor und Brom, $C_1$—$C_6$-Alkyl wie Methyl, Acylamino Nitro, Carboxy oder Carbamoyl substituiert sein kann; $C_1$—$C_6$-Alkoxy, insbesondere Methoxy; Acylamino, insbesondere Acetylamino und Benzoylamino; Carboxy; Nitro oder Carbamoyl substituiert sein können oder die Ringe X, X', T und T' können darüber hinaus durch eventuell substituierte anellierte Benzoringe zu Naphthalinsystemen erweitert sein können, wobei die Naphthalinringe durch Halogen wie Chlor und Brom, $C_1$—$C_6$-Alkyl wie Methyl, Acylamino wie Acetylamino oder Benzoylamino, Nitro, Carboxy oder Carbamoyl substituiert sein können.

2. Symmetrisches Azinpigment der Formel

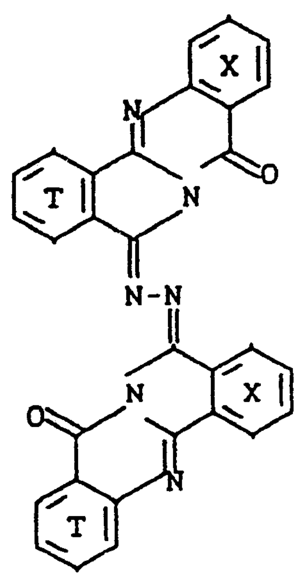

(II)

in der die mit X und/oder die mit T markierten Ringe durch 1, 2, 3 oder 4-Chlor- oder Bromatome substituiert sind.

3. Symmetrisches Azinpigmente der Formel (II) gemäß Anspruch 2, bei dem die mit X und/oder die mit T markierten Ringe einen Substituenten aus der Reihe Methyl, Methoxy, Acetylamino, Benzoylamino, Carboxy, Carbamoyl oder Nitro tragen.

4. Azinpigment der Formel

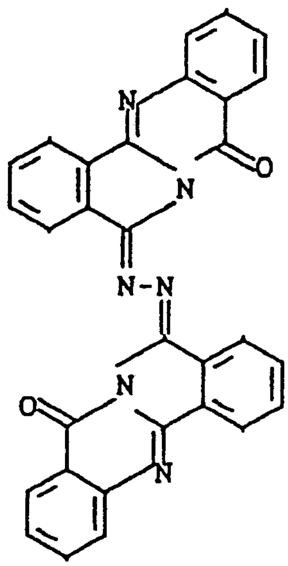

(III)

5. Azinpigment gemäß Anspruch 1, dadurch erhältlich, daß man eine Verbindung der Formel

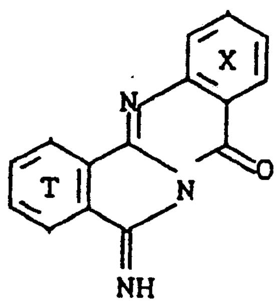

(IV)

mit einer Verbindung der Formel

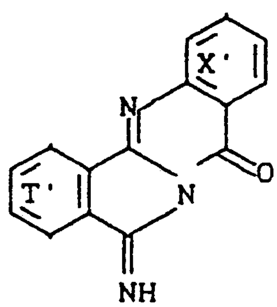

(IV a)

wobei T, T', X und X' die in Anspruch 1 angegebenen Bedeutungen haben, in Gegenwart von Hydrazin oder einer Hydrazin liefernden Verbindung umsetzt.

6. Azinpigmente gemäß Anspruch 1, dadurch erhältlich, daß man eine Verbindung der Formel

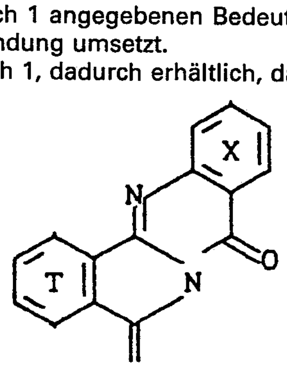

(V)

mit einer Verbindung der Formel

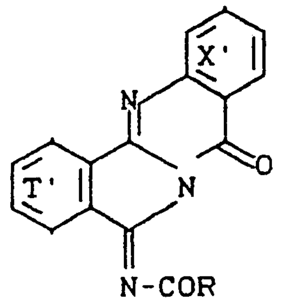

(V a)

wobei T, T', X und X' die in Anspruch 1 angegebene Bedeutung besitzen und R Alkyl oder Aryl bezeichnet, in Gegenwart von Hydrazin oder einer Hydrazin liefernden Verbindung umsetzt.

7. Azinpigmente gemäß Anspruch 1, dadurch erhältlich, daß man eine Verbindung der Formel

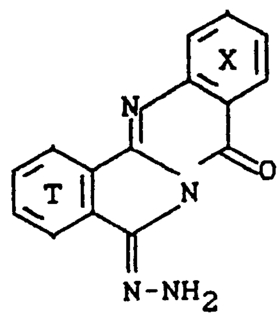

(IX)

mit einer Verbindung der Formel

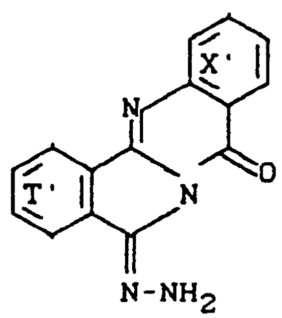

(IX a)

im sauren Medium unter Abspaltung von Hydrazin umsetzt.

8. Azinpigmente gemäß den Ansprüchen 5, 6 und 7, dadurch erhältlich, daß man die Verbindungen der Formeln (IV), (IVa), (V), (Va), (IX) und (IXa) durch Umsetzung eines Isatosäureanhydrids der Formel

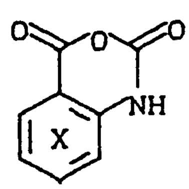

(VI) bzw. der Formel

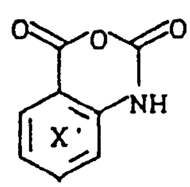

(VI a)

oder eines Anthranilsäureesters oder -amids der Formel

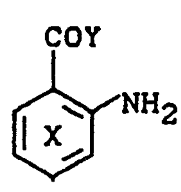

(VII) bzw. der Formel

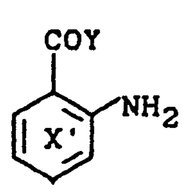

(VII a)

wobei X und X' die in Anspruch 1 angegebenen Bedeutungen haben und Y $—NH_2$ oder —OR' bezeichnet,

wobei R' für den Kohlenwasserstoffrest eines Esters steht, mit mindestens einem Mol einer Verbindung der Formel

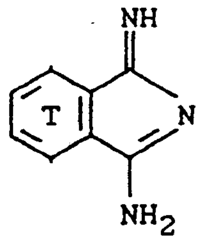 (VIII) bzw. der Formel 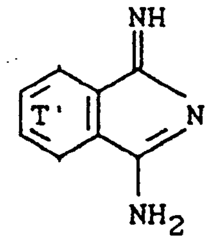 (VIII a)

wobei T und T' die in Anspruch 1 angegebenen Bedeutungen haben, in einem inerten organischen Lösungsmittel bei erhöhter Temperatur mit gegebenenfalls anschließender Acylierung oder Hydrazonbildung herstellt.

9. Verfahren zum Pigmentieren organischer makromolekularer Stoffe, dadurch gekennzeichnet, daß man Azinpigmente gemäß den Ansprüchen 1 bis 9 verwendet.

**Revendications**

1. Pigments aziniques de formule

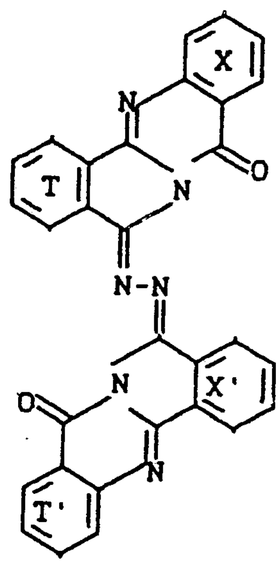

(I)

dans laquelle les noyaux désignés par les lettres X, X', T et T' peuvent être substitués chacun par 1, 2, 3 ou 4 substituants de la série comprenant un halogène, notamment le chlore et le brome; un groupe alkyle en $C_1$ à $C_6$, notamment le groupe méthyle; un groupe aryle, notamment le groupe phényle qui peut être substitué, quant à lui, par un halogène tel que le chlore et le brome, un groupe alkyle en $C_1$ à $C_6$ tel que le groupe méthyle, un groupe acylamino, nitro, carboxy ou carbamoyle; un groupe alkoxy en $C_1$ à $C_6$, notamment le groupe méthoxy; un groupe acylamino, notamment les groupes acétylamino et benzoylamino; un groupe carboxy; un groupe nitro ou carbamoyle, ou bien les noyaux X, X', T et T' peuvent en outre être élargis en systèmes naphthaléniques par des noyaux benzéniques condensés éventuellement substitués, les noyaux naphthaléniques pouvant être substitués par un halogène tel que le chlore et le brome, un groupe alkyle en $C_1$ à $C_6$ tel que le groupe méthyle, un groupe acylamino tel que le groupe acétylamino ou benzoylamino, un groupe nitro, carboxy ou carbamoyle.

2. Pigment azinique symétrique de formule

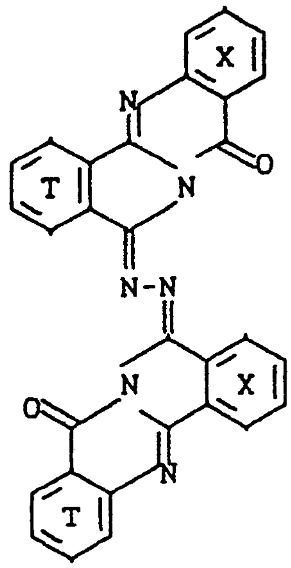

(II)

dans laquelle les noyaux marqués par la lettre X et/ou la lettre T sont substitués par 1, 2, 3 ou 4 atomes de chlore ou de brome.

3. Pigments aziniques symétriques de formule (II) suivant la revendication 2, dans lesquels les noyaux marqués de la lettre X et/ou de la lettre T portent un substituant de la série méthyle, méthoxy, acétylamino, benzoylamino, carboxy, carbamoyle ou nitro.

4. Pigment azinique de formule

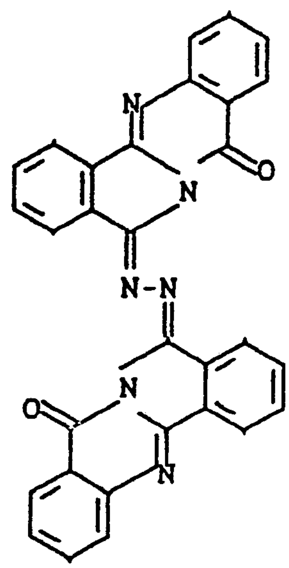

(III)

5. Pigment azinique suivant la revendication 1, pouvant être obtenu par réaction d'un composé de formule

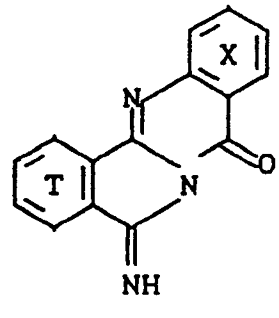

(IV)

avec un composé de formule

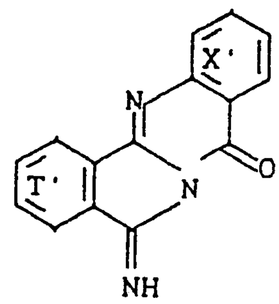

(IV a)

où T, T', X et X' ont les définitions indiquées dans la revendication 1, en présence d'hydrazine ou d'un composé libérant de l'hydrazine.

6. Pigments aziniques suivant la revendication 1, pouvant être obtenus par réaction d'un composé de formule

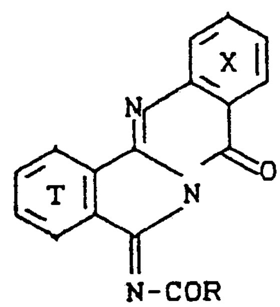

(V)

avec un composé de formule

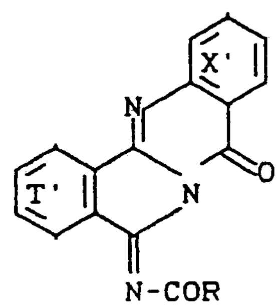

(V a)

où T, T', X et X' ont les définition indiquée dans la revendication 1 et R désigne un groupe alkyle ou aryle, en présence d'hydrazine ou d'un composé libérant de l'hydrazine.

7. Pigments aziniques suivant la revendication 1, pouvant être obtenus par réaction d'un composé de formule

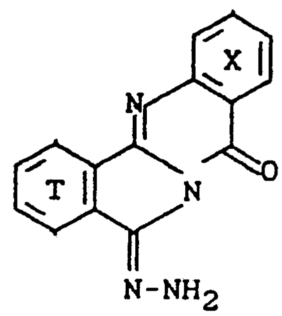

(IX)

avec un composé de formule

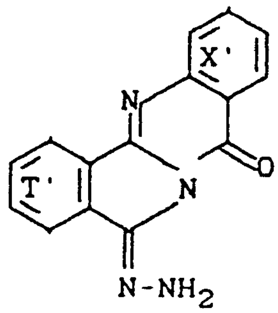

(IX a)

en milieu acide avec libération d'hydrazine.

8. Pigments aziniques suivant les revendications 5, 6 et 7, pouvant être obtenus par la fait que l'on prépare les composés de formules (IV), (IVa), (V), (Va), (IX) et (IXa) par réaction d'un anhydride d'acide insatique de formule

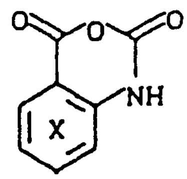

(VI)

ou de formule

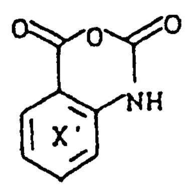

(VI a)

ou d'un ester ou amide d'acide anthranilique de formule

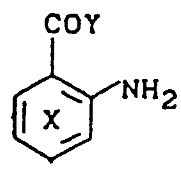

(VII)

et, respectivement, de formule

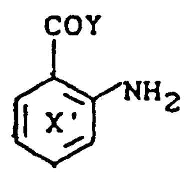

(VII a)

où X et X' ont les définitions indiquées dans la revendication 1 et Y désigne un groupe $-NH_2$ ou $-OR'$, R' représentant le reste hydrocarboné d'un ester, avec au moins une mole d'un composé de formule

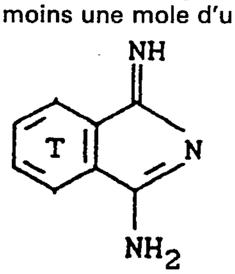

(VIII)

ou de formule

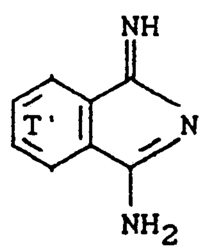

(VIII a)

où T et T' ont les définitions indiquées dans la revendication 1, dans un solvant organique inerte à température élevée, la réaction étant suivie, le cas échéant, d'une acylation ou de la formation d'hydrazone.

9. Procédé de pigmentation de substances organiques macromoléculaires, caractérisé en ce qu'on utilise des pigments aziniques suivant les revendications 1 à 9.

## Claims

1. Azine pigments of the structure

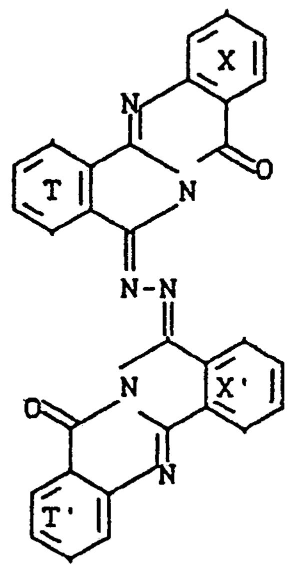

(I)

wherein the rings designated by X, X', T and T' can be substituted in each case by 1, 2, 3 or 4 substituents from the series comprising halogen, in particular chlorine and bromine; $C_1$—$C_6$-alkyl, in particular methyl; aryl, in particular phenyl which in turn can be substituted by halogen, such as chlorine and bromine, $C_1$—$C_6$-alkyl, such as methyl, acylamino, nitro, carboxyl or carbamoyl; $C_1$—$C_6$-alkoxy, in particular methoxy; acylamino, in particular acetylamino and benzoylamino; carboxyl; nitro or carbamoyl; or the rings X, X', T and T' can furthermore be expanded to naphthalene systems by means of fused benzo rings, which may be substituted, and the naphthalene rings can be substituted by halogen, such as chlorine and bromine, $C_1$—$C_6$-alkyl, such as methyl, acrylamino, such as acetylamino or benzoylamino, nitro, carboxyl or carbamoyl.

2. Symmetric azine pigment of the formula

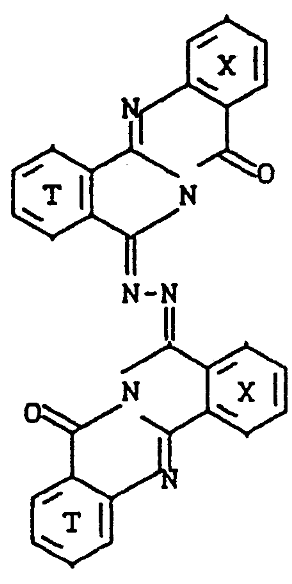

(II)

in which the rings denoted by X and/or the rings denoted by T are substituted by 1, 2, 3 or 4 chlorine or bromine atoms.

3. Symmetric azine pigments of the formula (II) according to Claim 2, in which the rings denoted by X and/or the rings denoted by T carry a substituent from the series comprising methyl, methoxy, acetylamino, benzoyl-amino, carboxyl, carbamoyl and nitro.

4. Azine pigment of the formula

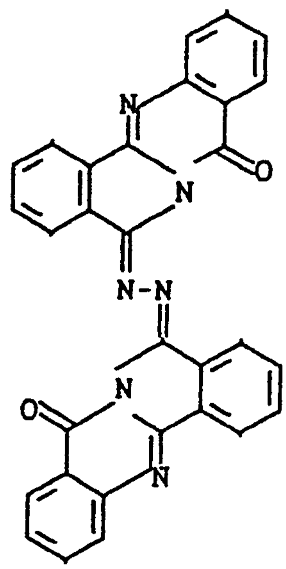

(III)

5. Azine pigment according to Claim 1, which can be obtained if a compound of the formula

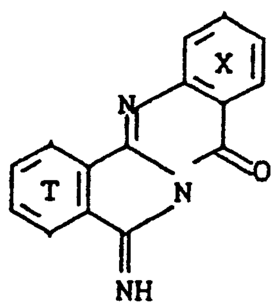

(IV)

is reacted with a compound of the formula

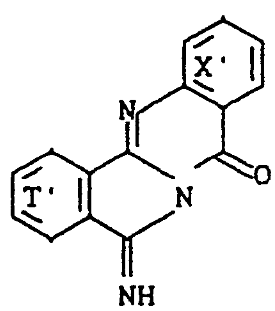

(IV a)

wherein

T, T', X and X' have the meanings given in Claim 1, in the presence of hydrazine or, of a hydrazine-donating compound.

6. Azine pigments according to Claim 1, which can be obtained if a compound of the formula

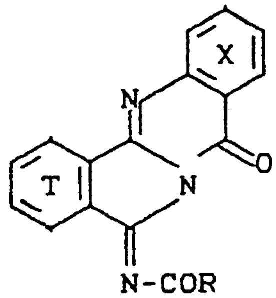

(V)

is reacted with a compound of the formula

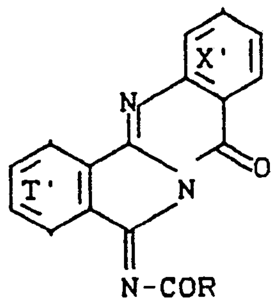

(V a)

wherein

T, T', X and X' have the meanings given in Claim 1 and

R designates alkyl or aryl, in the presence of hydrazine or of a hydrazine-donating compound.

7. Azine pigments according to Claim 1, which can be obtained if a compound of the formula

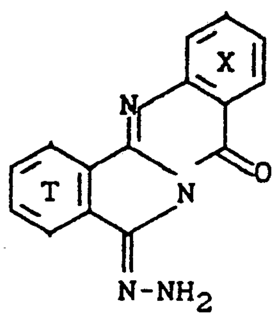

(IX)

is reacted with a compound of the formula

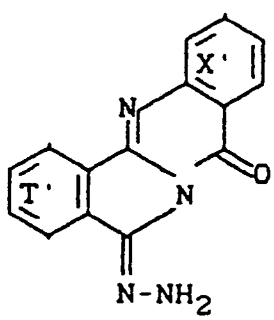

(IX a)

in an acidic medium, with elimination of hydrazine.

8. Azine pigments according to Claims 5, 6 and 7, obtainable by a method in which the compounds of

the formulae (IV), (IV a), (V), (Va), (IX) and (IXa) are prepared by reacting an isatoic anhydride of the formula

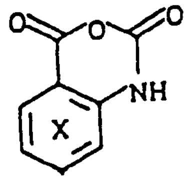 (VI) or of the formula 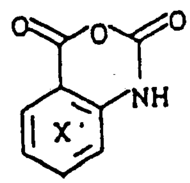 (VIa)

or an anthranilic acid ester or anthranilic acid amide of the formula

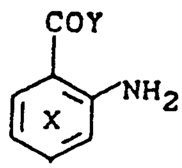 (VII) or of the formula 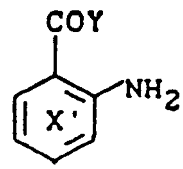 (VIIa)

wherein
X und X′ have the meanings given in Claim 1 and
Y designates $-NH_2$ or $-OR'$,
wherein
R′ represents the hydrocarbon radical of an ester, with at least one mol of a compound of the formula

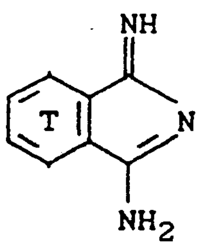 (VIII) or of the formula 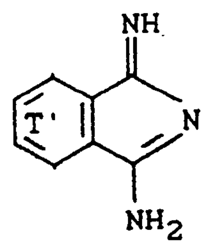 (VIII a)

wherein
T and T′ have the meanings given in Claim 1, in an inert organic solvent at elevated temperature with, if necessary, subsequent acylation or hydrazone formation.

9. Process for pigmenting organic macromolecular substances, characterised in that azine pigments according to Claims 1 to 9 are used.